# EUROPÄISCHE PATENTSCHRIFT

(11) **EP 2 417 087 B1**
(45) Veröffentlichungstag und Bekanntmachung des Hinweises auf die Patenterteilung: **13.03.2013**
(21) Anmeldenummer: 10711395.3
(22) Anmeldetag: 29.03.2010
(51) Int. Cl.: C07C 29/149, C07C 29/50, C07C 45/33, C07C 51/31, C07C 67/08

(54) **VERFAHREN ZUR HERSTELLUNG VON 1,6-HEXANDIOL DURCH HYDRIERUNG VON OLIGO- UND POLYESTERN**
METHOD FOR PRODUCING 1,6-HEXANEDIOL BY HYDROGENATION OF OLIGO- AND POLYESTERS
PROCÉDÉ DE PRODUCTION DE 1,6-HEXANEDIOL PAR HYDROGÉNATION D'OLIGOESTERS ET DE POLYESTERS

(30) Priorität: 08.04.2009 DE 102009002280
(43) Veröffentlichungstag der Anmeldung: 15.02.2012
(60) Teilanmeldung: 12192908.7
(73) Patentinhaber: BASF SE, 67056 Ludwigshafen (DE)
(72) Erfinder: ABILLARD, Olivier, 68161 Mannheim (DE); PINKOS, Rolf, 67098 Bad Dürkheim (DE); TEBBEN, Gerd-Dieter, Dr., 68239 Mannheim (DE); SIRCH, Tilman, 67105 Schifferstadt (DE); URBANCZYK, Daniel, 64283 Darmstadt (DE); URTEL, Heiko, 67240 Bobenheim-Roxheim (DE); TOMPERS, Rolf, 68259 Mannheim (DE)
(86) Internationale Anmeldenummer: PCT/EP2010/054114
(87) Internationale Veröffentlichungsnummer: WO 2010/115759

(56) Entgegenhaltungen:
- EP-A1- 0 922 688
- EP-A2- 0 721 928
- WO-A1-2004/085356
- DE-B- 1 255 650
- US-A- 3 524 892
- US-A- 3 933 930

## Beschreibung

Die vorliegende Erfindung betrifft ein Verfahren zur Hydrierung von Oligo- und/oder Polyestern, erhältlich durch Veresterung einer Dicarbonsäure-Lösung (DCL) mit einem Diol oder Diolgemisch, wobei die Hydrierung in Gegenwart eines Katalysators durchgeführt wird, dessen Katalysator-Vorläufer Kupferoxid, Aluminiumoxid und mindestens ein Oxid des Lanthans, Eisen, Wolframs, Molybdäns, Titans oder Zirkoniums enthält sowie ein Verfahren zur Herstellung von 1,6-Hexandiol durch katalytische Hydrierung von Estergemischen, die als Hauptkomponenten Oligo- und Polyester der Adipinsäure und 6-Hydroxycapronsäure enthalten und durch Veresterung von DCL mit Diolen und dabei insbesondere 1,6-Hexandiot oder Diolgemischen gewonnen werden.

Es besteht ein großer Bedarf an 1,6-Hexandiol, das einen gesuchten Monomerbaustein darstellt, der überwiegend auf dem Polyester- und Polyurethan-Gebiet eingesetzt wird.

H.-J. Arpe beschreibt in Industrielle organische Chemie, 6. Auflage 2007, WILEY-VCH-Verlag, Seite 267, vorletzter Absatz die Veresterung von Adipinsäure sowie die katalytische Hydrierung der Diester zu 1,6-Hexandiol.

Aus WO 2004/085356 ist die Hydrierung von Adipinsäuredimethylester, der aus Adipinsäure und Methanol als Monoalkohol hergestellt wurde, zu 1,6-Hexandiol bekannt. Der verwendete Festbett-Katalysator enthielt Kupfer, Aluminiumoxid und Lanthanoxid. Hydriert wurde bei 240 °C/200 bar. Laut Beispiel 2 wurden Ester-Umsätze von 98 bis 99 % erzielt. Im Hydrieraustrag waren 1,6-Hexandiol-Anteile von 57 Gew.-% bzw. 62 Gew.-% enthalten. 62 Gew.-% entspricht etwa einer 1,6-Hexandiol-Ausbeute von 96 %. Nachteilig ist die Verwendung von reiner Adipinsäure wegen ihres Preises und die aufwendige Abtrennung von großen Mengen Methanol aus dem Hydrieraustrag. Außerdem treten Methanol-Verluste durch Dimethylether-Bildung auf. Die Hydrierung von Oligo- und/oder Polyestern der Adipinsäure und 6-Hydroxycapronsäure wird nicht beschrieben.

Anstelle von reiner Adipinsäure wurde auch schon Dicarbonsäurelösung (DCL) als Ausgangsprodukt für die Herstellung von 1,6-Hexandiol verwenden. Sie fällt bei der Oxidation von Cyclohexan mit Luft zu Cyclohexanon und Cyclohexanol durch Extraktion des Oxidats mit Wasser als Abfallprodukt an.

Der Verbund mit Produktionsanlagen zur Herstellung von Cyclohexanol und Cyclohexanon und die Nutzung des Abfallprodukts DCL als Ausgangsprodukt für die Herstellung von 1,6-Hexandiol führt gegenüber reiner Adipinsäure zu günstigen Einsatzstoffkosten. Sie stellt außerdem eine umweltfreundliche Nutzung eines Abfallprodukts dar.

Im Ausführungsbeispiel von DE 196 07 954 ist beschrieben, entwässerte Dicarbonsäure mit Methanol zu verestern und das Estergemisch nach Abtrennung von Leichtsiedern in Gegenwart von Kupfer, Zinkoxid und Aluminiumoxid enthaltenden Katalysatoren zu 1,6-Hexandiol und Methanol zu hydrieren. Bei 220°C und 220 bar betrug der Esterumsatz 99,5 %, die 1,6-Hexandiol-Selektivität > 99 %.

Nachteilig ist die Verwendung von Methanol, das abgetrennt und zurückgeführt werden muss und die im Lauf der Hydrierung abnehmende Seitendruckfestigkeit des Hydrierkatalysators.

Die Verwendung von Diolen, insbesondere von alpha, omega-Diolen als Veresterungsalkoholen stellt einen Vorteil gegenüber Monoalkoholen dar. Die Verluste an Veresterungsalkohol verringern sich und die Aufarbeitung des Hydrieraustrags wird vereinfacht.

EP-A 922 688 beschreibt die Hydrierung von Oligoestern, die aus Adipinsäure und 1,6-Hexandiol hergestellt wurden. Hydriert wurde in Gegenwart von Zinkoxid-freien Kupfer-katalysatoren, die Mangan- und Aluminiumoxid und mindestens ein Oxid von Metallen der VI. Nebengruppe enthielten. Die Hydrierung nach Beispiel 2 wurde bei 230°C und 300 bar durchgeführt. Der 1,6-Hexandiol-Gehalt des Hydrieraustrags betrug 97,1 %. Die Verwendung von DCL als Ausgangsprodukt für die Herstellung der Oligoester ist in EP-A 922 688 nicht beschrieben.

Der bisher genannte Stand der Technik zeigt, dass sich Adipinsäurediester, hergestellt aus reiner Adipinsäure und Monoalkoholen, DCL und Monoalkoholen und Oligo- und Polyestern, hergestellt aus reiner Adipinsäure und reinem 1,6-Hexandiol mit hohen Ausbeuten von etwa 96 bis 99 % zu 1,6-Hexandiol hydrieren lassen. Für die Hydrierung wurden dabei Katalysatoren auf der Basis von Kupfer und Zinkoxid, Kupfer und Aluminiumoxid oder Kupfer, Aluminiumoxid und Zinkoxid verwendet.

Es ist auch schon bekannt, ausgehend von DCL und 1,6-Hexandiol oder DiolGemischen hergestellte Oligo- und/oder Polyester katalytisch zu 1,6-Hexandiol zu hydrieren. Diese Variante besitzt den prinzipiellen Vorteil, dass das Zielprodukt 1,6-Hexandiol oder überwiegend 1,6-Hexandiol enthaltende Diolgemische aus dem Verfahren für die Veresterung der Dicarbonsäure verwendet werden können. Die für die Hydrierung der Oligo- und Polyester verwendeten Katalysatoren und/oder die erzielten 1,6-Hexandiol-Ausbeuten sind jedoch noch unbefriedigend.

So ist aus US 3.524.892 bekannt, Cyclohexan mit Luft zu einem Gemisch aus Cyclohexanon, Cyclohexanol, nicht umgesetztem Cyclohexan, Adipinsäure und 6-Hydroxycapronsäure zu oxidieren und das Reaktionsprodukt der Oxidation mit Wasser zu extrahieren. Durch Phasentrennung erhält man eine organische und eine wässrige Phase. Die organische Phase besteht überwiegend aus Cyclohexan, Cyclohexanol und Cyclohexanon und wird zumindest teilweise in die Oxidationsstufe zurückgeführt. Die wässrige Phase enthält überwiegend Adipinsäure und 6-Hydroxycapronsäure. Sie wird entwässert und mit reinem 1,6-Hexandiol zu Oligoestern umgesetzt. Die Oligoester werden bei 260 bis 275 °C und 275 bis 330 bar in Gegenwart von Kupferchromit-Katalysatoren zu 1,6-Hexandiol als Hauptprodukt hydriert.

Nachteilig ist die Verwendung von Katalysatoren, die wie Kupferchromit wegen ihres Chromgehalts toxisch sind und einen hohen Aufwand beim Betrieb der Hydrierung und bei der Entsorgung verbrauchter Katalysatoren und chromhaltiger Nebenprodukte erforderlich machen. Außerdem dokumentieren die hohe Hydriertemperatur und der hohe Hydrierdruck einen Mangel an Katalysator-Aktivität, die zu verstärkter Nebenprodukt-Bildung führt. Die Ausbeute an 1,6-Hexandiol nach diesem Verfahren wird nicht beschrieben.

EP-A 661255 beschreibt die Hydrierung von Estergemischen, die durch Veresterung von DCL mit 1,6-Hexandiol oder mit Diolgemischen, die bei der Hydrierung erhalten werden, hergestellt wurden. Die Hydrierung erfolgte diskontinuierlich bei 250 bis 300°C und 150 bis 300 bar. Als Katalysatoren wurden Kupfer und Zinkoxid enthaltende Suspensionskatalysatoren verwendet. In den Ausführungsbeispielen wurde bei 280°C und 280 bar hydriert. Angaben zu 1,6-Hexandiol-Ausbeuten, Ester-Umsätzen oder Katalysator-Verbrauchszahlen werden nicht beschrieben.

Nachteilig sind die hohe Hydriertemperatur, der hohe Hydrierdruck und die schwierige Abtrennung des suspendierten Hydrierkatalysators aus dem Hydrieraustrag.

JP 2005/008586 unterscheidet sich von EP-A 661 255 vor allem dadurch, dass Festbettkatalysatoren eingesetzt werden. Als Festbett-Katalysatoren werden CuO/ZnO-, CuO/ZnO/Al₂O₃-, CuO/SiO₂-, CuO/ZrO₂- und CuO/Cr₂O₃-Katalysatoren verwendet, wie es auf Seite 11, Abschnitt [0018] beschrieben ist. Dies sind Katalysatoren wie sie auch schon mit hohen Ausbeuten für die Hydrierung von Adipinsäureestern, hergestellt aus reiner Adipinsäure und Monoalkoholen, Estern von DCL mit Monoalkoholen und Oligo-und Polyestern, hergestellt aus reiner Adipinsäure und reinem 1,6-Hexandiol, verwendet wurden. Hydriert wurde bei 190 bis 250 °C und 1 bis 10 MPa. Eine Steigerung des Drucks führt zu keiner Verbesserung des Reaktionsergebnisses wie auf Seite 13, in Abschnitt [0022], Zeilen 3 bis 4 beschrieben.

Auf Seite 15, Abschnitt [0026] wird die Herstellung des Oligo-/Polyestergemisch wie folgt beschrieben: Von Cyclohexanol und Cyclohexanon befreites Produkt aus der Flüssigphasen-Oxidation von Cyclohexan wurde mit wässriger Natronlauge gereinigt. Die dabei abgetrennte wässrige Lösung von Natriumcarboxylaten wurde mit Schwefelsäure angesäuert. Die freigesetzten Carbonsäuren wurden mit Methyl-i-butylketon extrahiert. Das nach Abtrennen des Extraktionsmittels erhaltene Carbonsäure-Gemisch, das nicht mehr der nach Oxidation und Extraktion mit Wasser erhaltenen DCL entspricht, da unter anderem folgende Produkte wie Cyclohexanol, 1,2- und 1,4-Cyclohexandiol, Cyclohexanon, Cyclohexandion sowie niederwertige Alkohole entfernt wurden, wurde mit einem Gemisch aus 1,6-Hexandiol, 1,5-Pentandiol und einwertigen Alkoholen wie Pentanol und Butanol verestert.

Nachteilig an JP 2005/008586 ist, dass nach den Ausführungsbeispielen 1 bis 5 (Beispiel 1 bis 3 und 5 CuO/ZnO, Beispiel 4 CuO/SiO₂ als Katalysator) lediglich 1,6-Hexandiol-Ausbeuten zwischen 84 und 89 % bei Umsätzen von nur 84 bis 91 % erzielt wurden. Weiterhin, dass die genannten 1,6-Hexandiol-Ausbeuten nur dann erreicht werden, wenn die Hydrierung in Gegenwart eines niedrigwertigen Monoalkohols, wie Methanol, durchgeführt wurde. Wenn kein niedrigwertiger Monoalkohol verwendet wird, ist die 1,6-Hexandiol-Ausbeute gering wie auf Seite 12, Abschnitt [0019], die drei letzte Zeilen, beschrieben.

Bei der Veresterung einer DCL mit Diolen oder Diolgemischen entstehen oligomere Ester unterschiedlicher Zusammensetzung. Deren Hydrierung stellt hohe Ansprüche an den entsprechenden Katalysator, da dieser nicht vergiften darf und trotzdem eine hohe Aktivität und Selektivtät zeigen muss. Ausgehend vom Stand der Technik ist es daher nicht naheliegend, dass Katalysatoren, die bei der Hydrierung von monomeren Estern, hergestellt durch die Umsetzung von reiner Adipinsäure mit niederwertigem Alkohol wie z. B. Methanol, auch die Hydrierung von Estern, die aus einer komplexen DCL durch Veresterung mit einem Diol oder Diolgemisch hergestellt wurden, zu ähnlich guten Ausbeuten und Reinheiten des Endproduktes führen.

Es bestand daher die Aufgabe, ein Verfahren zur Hydrierung von Oligo- und Polyestern erhältlich durch Veresterung einer DCL mit einem Diolgemisch in Gegenwart eines Katalysators bereit zu stellen. Der dabei für die Hydrierung verwendete Katalysator sollte eine hohe Hydrieraktivität besitzen, um einen praktisch vollständigen Oligo-und/oder Polyester-Umsatz bei hoher 1,6-Hexandiol-Ausbeute und Selektivität zu erzielen. Der Katalysator sollte weiterhin eine hohe Standzeit bei gleichbleibendem Umsatz und wenig verminderter Seitendruckfestigkeit zulassen. Weiterhin sollte er keine Nebenkomponenten bilden, die die Reinigung des 1,6-Hexandiols wesentlich komplizieren. Schließlich sollte er keine toxischen Komponenten enthalten, die das Arbeiten mit diesem Katalysator und seine Entsorgung erschweren.

Diese Aufgabe wird gelöst durch ein Verfahren zur Hydrierung von Oligo- und/oder Polyestern erhältlich durch Veresterung einer Dicarbonsäurelösung mit einem Diol oder Diolgemisch, wobei dem anfallenden Estergemisch eine Base, ausgewählt aus der Gruppe bestehend aus Lithium-, Kalium- und Natrium-Alkoholat, zudosiert wird, und wobei die Hydrierung mittels eines Katalysator-Formkörpers, dessen Vorläufer gemäß einem Verfahren herstellbar ist, in dem
(i) ein oxidisches Material, umfassend Kupferoxid, Aluminiumoxid und mindestens eins der Oxide des Lanthans, Wolframs, Molybdäns, Titans, Zirkoniums oder Eisen bereitgestellt wird,
(ii) dem oxidischen Material pulverförmiges metallisches Kupfer, Kupferblättchen, pulverförmiger Zement, Graphit oder ein Gemisch davon zugegeben wird
(iii) das aus (ii) resultierende Gemisch zu einem Formkörper verformt wird,
durchgeführt wird, wobei das oxidische Material erhältlich ist durch simultanes oder nacheinander Fällen der Aktivkomponente Kupfer, der Komponente Aluminium und der Komponente mindestens eines der Oxide des Lanthans, Wolframs, Molybdäns, Titans oder Zirkoniums und anschließendes Trocknen und Calcinieren und nach der Verformung nach Schritt (iii) der Katalysator-Formkörper nochmals calciniert wird.

Die vorliegende Erfindung betrifft ein Verfahren zur Hydrierung von Oligo-, und Polyestern der DCL zu Diolen unter Verwendung spezieller Katalysator-Formkörpervorläufer für die Hydrierung, wobei die Katalysator-Formkörpervorläufer neben Kupfer- und Aluminiumoxide wenigstens ein Oxide des Lanthan, Wolfram, Molybdäns, Titans, Zirkoniums oder Eisen sowie metallisches Kupfer, Kupferblättchen, pulverförmiger Zement, Graphit oder ein Gemisch enthalten. Die Veresterung der DCL erfolgt mittels Diolen, die ausgewählt sind aus der Gruppe von Glycerin, Trimethylolpropan, Propylenglykol, Ethylenglykol, 1,3-Propandiol, 1,4-Butandiol, 1,5-Pentandiol, 1,6-Hexandiol, 1,2-Cyclohexandiol, 1,4-Cyclohexandiol, 1,8-Octandiol, 1,10-Decandiol, 1,12-Dodecandiol oder Gemische dieser Diole. Bevorzugt sind 1,4-Butandiol, 1,5-Pentandiol, 1,6-Hexandiol, 1,2-Cyclohexandiol, 1,4-Cyclohexandiol oder Gemische dieser Diole. Bevorzugt ist die Veresterung der DCL mit Hilfe von 1,6- Hexandiol zu Oligo- und/oder Polyestern.

Von den Oxiden des Lanthans, Wolframs, Molybdäns, Titans oder Zirkoniums ist Lanthanoxid bevorzugt.

Unter Eisenoxid ist Eisen(III)oxid gemeint.

In bevorzugten Ausführungsformen werden die erfindungsgemäßen Formkörper als Voll,- Tränk-, Schalen- und Fällkatalysatoren eingesetzt.

Der in dem erfindungsgemäßen Verfahren verwendete Katalysator für die Hydrierung zeichnet sich dadurch aus, dass die Aktivkomponente Kupfer, die Komponente Aluminium und die Komponente mindestens eines der Oxide des Lanthans, Wolframs, Molybdäns, Titans oder Zirkoniums bevorzugt mit einer Sodalösung simultan oder nacheinander gefällt werden, im Anschluss getrocknet, calciniert , tablettiert und nochmals calciniert wird.

Insbesondere kommt folgende Fällungsmethode in Betracht:
A) Eine Kupfersalzlösung, eine Aluminiumsalzlösung und eine Lösung mindestens eines Salzes des Lanthans, Wolframs, Molybdäns, Titans oder Zirkoniums oder eine Lösung, enthaltend Kupfer-, Aluminium- und mindestens eines der Salze des Lanthans, Wolframs, Molybdäns, Titans oder Zirkoniums, wird parallel oder nacheinander mit einer Sodalösung gefällt. Das gefällte Material im Anschluss getrocknet und ggf. calciniert.
B) Fällung einer Kupfersalzlösung und einer Lösung mindestens eines Salzes des Lanthans, Wolframs, Molybdäns, Titans oder Zirkoniums oder einer Lösung, enthaltend Kupfersalz und mindestens ein Salz des Lanthans, Wolframs, Molybdäns, Titans oder Zirkoniums, auf einen vorgefertigten Aluminiumoxidträger. Dieser liegt in einer besonders bevorzugten Ausführungsform als Pulver in einer wässrigen Suspension vor. Das Trägermaterial kann aber auch als Kugeln, Stränge, Splitt oder Tabletten vorliegen.
B1) In einer Ausführungsform (I) wird eine Kupfersalzlösung und eine Lösung mindestens eines Salzes des Lanthans, Wolframs, Molybdäns, Titans oder Zirkoniums oder ein Lösung, enthaltend Kupfersalz und mindestens ein Salz des Lanthans, Wolframs, Molybdäns, Titans oder Zirkoniums, bevorzugt mit Sodalösung, gefällt. Als Vorlage wird eine wässrige Suspension des Trägermaterials Aluminiumoxid verwendet.

Ausgefällte Niederschläge, die aus A) oder B) resultieren, werden in üblicher Weise filtriert und vorzugsweise alkalifrei gewaschen, wie dies beispielsweise in der DE 198 09 418.3 beschrieben ist.

Sowohl die Endprodukte aus A) als auch die aus B) werden bei Temperaturen von 50 bis 150°C, vorzugsweise bei 120°C getrocknet und im Anschluß ggf. vorzugsweise 2 Stunden bei im allgemeinen 200 bis 600°C, insbesondere bei 300 bis 500°C calciniert.

Als Ausgangssubstanzen für A) und/oder B) können prinzipiell alle in den bei der Aufbringung verwendeten Lösungsmitteln löslichen Cu(I) und/oder Cu(II)-Salze, wie beispielsweise Nitrate, Carbonate, Acetate, Oxalate oder Ammonium-Komplexe, analoge Aluminiumsalze und Salze des Lanthans, Wolframs, Molybdäns, Titans oder Zirkoniums verwendet werden. Besonders bevorzugt für Verfahren gemäß A) und B) wird Kupfernitrat eingesetzt.

In dem erfindungsgemäßen Verfahren wird das oben beschriebene getrocknete und gegebenenfalls calcinierte Pulver bevorzugt zu Tabletten, Ringen, Ringtabletten, Extrudaten, Wabenkörpern oder ähnlichen Formkörpern verarbeitet. Hierfür sind sämtliche aus dem Stand der Technik geeigneten Verfahren denkbar.

Die Zusammensetzung des oxidischen Material ist im allgemeinen so beschaffen, dass der Anteil an Kupferoxid im Bereich von 40 bis 90 Gew.-%, der Anteil an Oxiden des Lanthans, Wolframs, Molybdäns, Titans oder Zirkoniums im Bereich von 0 bis 50 Gew.-% und der Anteil an Aluminiumoxid im Bereich bis zu 50 Gew.-%, jeweils bezogen auf das Gesamtgewicht der Summe der oben genannten oxidischen Bestandteile, liegt, wobei diese drei Oxide zusammen mindestens 80 Gew.-% des oxidischen Materials nach Calcinierung darstellen, wobei Zement nicht dem oxidischen Material in obigem Sinne zugerechnet wird.

In einer bevorzugten Ausführungsform betrifft die vorliegende Erfindung daher ein Verfahren, wie oben beschrieben, das dadurch gekennzeichnet ist, dass das oxidische Material
(a) Kupferoxid mit einem Anteil im Bereich von 50 ≤ x ≤ 80, vorzugsweise 55 ≤ x ≤ 75 Gew.-%,
(b) Aluminiumoxid mit einem Anteil im Bereich von 15 ≤ y ≤ 35, vorzugsweise 20 ≤ y ≤ 30 Gew.-% und
(c) mindestens eins der Oxide des Lanthans, Wolframs, Molybdäns, Titans oder Zirkoniums mit einem Anteil im Bereich von 2 ≤ z ≤ 20, bevorzugt 3 ≤ z ≤ 15 Gew.-%,
jeweils bezogen auf das Gesamtgewicht des oxidischen Materials nach Calcinierung, wobei gilt: 80 ≤ x + y + z ≤ 100, insbesondere 95 ≤ + y + z ≤ 100, umfasst.

Das erfindungsgemäße Verfahren und die eingesetzten Katalysatoren für die Hydrierung zeichnen sich dadurch aus, dass durch die Zugabe von Lanthan, Wolfram, Molybdän, Titan oder Zirkonium bei der Fällung zu einer hohen Stabilität des Formkörpers, der als Katalysator eingesetzt wird, führt.

Im allgemeinen wird dem oxidischen Material pulverförmiges Kupfer, Kupferblättchen oder pulverförmiger Zement oder Graphit oder ein Gemisch davon im Bereich von 1 bis 40 Gew.-%, bevorzugt im Bereich von 2 bis 20 Gew.-% und besonders bevorzugt im Bereich von 3 bis 10 Gew.-%, jeweils bezogen auf das Gesamtgewicht des oxidischen Materials, zugesetzt.

Als Zement wird vorzugsweise ein Tonerdezement eingesetzt. Besonders bevorzugt besteht der Tonerdezement im Wesentlichen aus Aluminiumoxid und Calciumoxid, und besonders bevorzugt besteht er aus ungefähr 75 bis 85 Gew.-% Aluminiumoxid und ungefähr 15 bis 25 Gew.-% Calciumoxid. Ferner kann ein Zement auf Basis Magnesiumoxid/Aluminiumoxid, Calciumoxid/Siliciumoxid und Calciumoxid/Aluminiumoxid/Eisenoxid verwendet werden.

Insbesondere kann das oxidische Material in einem Anteil von höchstens 10 Gew.-%, bevorzugt höchstens 5 Gew.-%, bezogen auf das Gesamtgewicht des oxidischen Materials, mindestens eine weitere Komponente aufweisen, die ausgewählt wird aus der Gruppe bestehend aus den Elementen Re, Fe, Ru, Co, Rh, Ir, Ni, Pd und Pt.

In einer weiter bevorzugten Ausführungsform des erfindungsgemäßen Verfahrens wird dem oxidischen Material vor dem Verformen zum Formkörper zusätzlich zu dem Kupferpulver, der Kupferblättchen oder dem Zementpulver oder dem Gemisch davon Graphit zugesetzt. Vorzugsweise wird soviel Graphit zugegeben, dass die Verformung zu einem Formkörper besser durchgeführt werden kann. In einer bevorzugten Ausführungsform werden 0,5 bis 5 Gew.-% Graphit, bezogen auf das Gesamtgewicht des oxidischen Materials, zugegeben. Dabei ist es gleichgültig, ob Graphit dem oxidischen Material vor oder nach oder gleichzeitig mit dem Kupferpulver, den Kupferblättchen oder dem Zementpulver oder dem Gemisch davon zugesetzt wird.

Demgemäss betrifft die vorliegende Erfindung auch ein Verfahren, wie oben beschrieben, das dadurch gekennzeichnet, ist, dass dem oxidischen Material oder dem aus (ii) resultierendem Gemisch Graphit in einem Anteil im Bereich von 0,5 bis 5 Gew.-%, bezogen auf das Gesamtgewicht des oxidischen Materials, zugegeben wird.

In einer bevorzugten Ausführungsform können in dem erfindungsgemäßen Verfahren als Katalysator-Vorläufer für die Hydrierung auch ein Formkörper eingesetzt werden, umfassend
ein oxidisches Material, das
(a) Kupferoxid mit einem Anteil im Bereich von 50 ≤ x ≤ 80, vorzugsweise 55 ≤ x ≤ 75 Gew.-%,
(b) Aluminiumoxid mit einem Anteil im Bereich von 15 ≤ y ≤ 35, vorzugsweise 20 ≤ y ≤ 30 Gew.-% und
(c) mindestens eins der Oxide des Lanthans, Wolframs, Molybdäns, Titans oder Zirkoniums mit einem Anteil im Bereich von 2 ≤ z ≤ 20, bevorzugt 3 bis 15 Gew.-%,
jeweils bezogen auf das Gesamtgewicht des oxidischen Materials nach Calcinierung, wobei gilt: 80 ≤ x + y + z ≤ 100, insbesondere 95 ≤ x + y+ z ≤ 100 umfasst,
metallisches Kupferpulver, Kupferblättchen oder Zementpulver oder ein Gemisch davon mit einem Anteil im Bereich von 1 bis 40 Gew.-%, bezogen auf das Gesamtgewicht des oxidischen Materials, und
Graphit mit einem Anteil von 0,5 bis 5 Gew.-%, bezogen auf das Gesamtgewicht des oxidischen Materials,
wobei die Summe der Anteile aus oxidischem Material, metallischem Kupferpulver, Kupferblättchen oder Zementpulver oder einem Gemisch davon und Graphit mindestens 95 Gew.-% des Formkörpers ergeben.

Nach Zugabe des Kupferpulvers, der Kupferblättchen oder des Zementpulvers oder des Gemischs davon und gegebenenfalls Graphit zu dem oxidischen Material wird der im Anschluss an die Verformung erhaltene Formkörper gegebenenfalls mindestens einmal calciniert über eine Zeit von im allgemeinen 0,5 bis 10 h, bevorzugt 0,5 bis 2 Stunden. Die Temperatur bei diesem mindestens einen Calcinierschritt liegt im allgemeinen im Bereich von 200 bis 600°C, bevorzugt im Bereich von 250 bis 500°C und besonders bevorzugt im Bereich von 270 bis 400°C.

Im Falle der Formgebung mit Zementpulver kann es vorteilhaft sein, den vor der Calcinierung erhaltenen Formkörper mit Wasser zu befeuchten und anschließend zu trocknen.

In einer weiteren Ausführung kann der erhaltene Formkörper auch noch mit siedendem Wasser und/oder Dampf behandelt werden, bevor er für die Hydrierung eingesetzt wird.

Bei Einsatz als Katalysator in der oxidischen Form wird der Formkörper vor Beschickung mit der Hydrierlösung mit reduzierenden Gasen, beispielsweise Wasserstoff, vorzugsweise Wasserstoff-Inertgasgemischen, insbesondere Wasserstoff/Stickstoffgemischen bei Temperaturen im Bereich von 100 bis 500°C, bevorzugt im Bereich von 150 bis 350°C und insbesondere im Bereich von 180 bis 200°C vorreduziert. Bevorzugt wird dabei ein Gemisch mit einem Wasserstoffanteil im Bereich von 1 bis 100 Vol.-%, besonders bevorzugt im Bereich von 1 bis 50 Vol.-% verwendet.

In einer bevorzugten Ausführungsform wird der erfindungsgemäße Formkörper vor dem Einsatz als Katalysator in an sich bekannter Weise durch Behandlung mit reduzierenden Medien aktiviert. Das Aktivieren erfolgt entweder vorab in einem Reduktionsofen oder nach dem Einbau im Reaktor. Ist der Reaktor vorab im Reduktionsofen aktiviert worden, wird er in den Reaktor eingebaut und direkt unter Wasserstoffdruck mit der Hydrierlösung beschickt.

Ein weiterer Gegenstand des Verfahrens ist ein Verfahren zur Herstellung von 1,6-Hexandiol umfassend folgende Schritte
a) Oxidation von Cyclohexan mit Sauerstoff oder Sauerstoff enthaltenden Gasen zu Gemischen aus Cyclohexanol, Cyclohexanon und Carbonsäuren mit bis zu sechs Kohlenstoffatomen,
b) Umsatz des nach a) erhaltene Reaktionsgemisches mit Wasser und Abtrennung der DCL aus dem flüssigen zweiphasigen Reaktionsgemisch,
c) Veresterung der aus b) erhaltenen DCL mit einem Alkohol,
d) katalytische Hydrierung des aus c) erhaltenen Estergemisches und
e) Destillation des aus d) erhaltenen Hydrieraustrags zu 1,6-Hexandiol,
dadurch gekennzeichnet, dass man
die Veresterung in c) mit mindestens einem Diol mit zwei bis zwölf Kohlenstoffatomen durchführt,
dem in c) anfallenden Estergemisch eine Base, ausgewählt aus der Gruppe bestehend aus Lithium-, Kalium-, und Natrium-Alkoholat, zudosiert, und
das in c) erhaltene Veresterungsgemisch in der Flüssigphase in Gegenwart eines Katalysator-Formkörpers gemäß Schritt d) hydriert, dessen Vorläufer erhältlich ist durch
(i) Bereitstellung eines oxidischen Material nach erster Calcinierung enthaltend Kupferoxid, Aluminiumoxid und mindestens eins der Oxide des Lanthans, Wolframs, Molybdäns, Titans, Zirkoniums oder Eisen,
(ii) Zugabe von pulverförmigem metallischem Kupfer, Kupferblättchen, pulverförmigem Zement, Graphit oder einem Gemisch zu dem oxidischen Material in aus Schritt i),
(iii) Formung des aus (ii) resultierenden Gemisches zu einem Formkörper und
(iv) zweiter Calcinierung des aus Schritt (iii) erhaltenen Formkörpers.

Dieses erfindungsgemäße Verfahren zur Herstellung von 1,6-Hexandiol als auch das erfindungsgemäße Verfahren zur Hydrierung von Oligo- und/oder Polyerstern verwendet als Ausgangsmaterial die wässrigen Lösungen von Carbonsäuren, die bei der Oxidation von Cyclohexan zu Cyclohexanol und Cyclohexanon (vgl. Ullmann's Encyclopedia of Industrial Chemistry, 6. Ed., Vol. 10, S. 282, Figure 2) als Nebenprodukte entstehen, im Folgenden Dicarbonsäurelösung (DCL) genannt. Diese Dicarbonsäurelösungen enthalten (berechnet wasserfrei in Gew.-%) im Allgemeinen zwischen 10 und 40 % Adipinsäure, zwischen 10 und 60 % 6-Hydroxycapronsäure, zwischen 1 und 10 % Glutarsäure, zwischen 1 und 10 % 5-Hydroxyvaleriansäure, zwischen 0,5 und 5 % 5-Formylvaleriansäure, zwischen 1 und 5 % 1,2-Cyclohexandiole, zwischen 1 und 5 % 1,4-Cyclohexandiole, zwischen 2 und 10 % Ameisensäure sowie eine Vielzahl weiterer Mono- und Dicarbonsäuren, Ester, Oxo- und Oxa-Verbindungen, deren Einzelgehalte im Allgemeinen 5 % nicht übersteigen. Beispielsweise seien Essigsäure, Propionsäure, Buttersäure, Valeriansäure, Capronsäure, Oxalsäure, Malonsäure, Dihydromuconsäure, Bernsteinsäure, 4-Hydroxybuttersäure, gamma-Butyrolacton und Caprolacton als Mono- und dicarbonsäuren, Ester, Oxo- und Oxa-Verbindungen genannt.

Eine genauere Analyse der DCL ergab als weitere Inhaltsstoffe 1,4-Cyclohexandion und 4-Hydroxycyclohexanon in Mengen von 0,01 bis 2 Gew.-%.

Die DCL ist im Allgemeinen eine wässrige Lösung mit einem Wasseranteil von 20 bis 80 Gew.-%.

Von den Inhaltsstoffen der DCL können Adipinsäure, 5-Formylvaleriansäure, 6-Hydroxycapronsäure, Dihydromuconsäure und Caprolacton durch Hydrierung in 1,6-Hexandiol überführt werden. Um reines 1,6-Hexandiol zu erhalten, müssen alle anderen Komponenten während der Verfahrensschritte c) bis e) abgetrennt werden.

Aldehyde wie 5-Formylvaleriansäure und Ketone wie 1,4-Cyclohexandion und 4-Hydroxycyclohexanon können bei der späteren Veresterung mit Diolen Acetale und Ketale bilden. Hierdurch kann 5-Formylvaleriansäure durch Folgereaktionen der Acetale für die Herstellung von 1,6-Hexandiol verloren gehen. Durch die Acetal- oder Ketalbildung kann der jeweils gebundene Alkohol ganz oder teilweise verloren gehen.

Je nach Zusammensetzung der DCL kann es daher vorteilhaft sein, die enthaltenen Aldehyde und Ketone vor dem Veresterungsschritt c) katalytisch zu Alkoholen zu hydrieren.

Wurde die Cyclohexan-Oxidation in Abwesenheit eines Deperoxidations-Katalysators, wie beispielsweise Cobaltnaphthenat, durchgeführt, so enthält die DCL wie in DE-A 1 951 250 und EP-A 847 979 beschrieben 6-Hydroperoxycapronsäure. Wurde in Anwesenheit eines Deperoxidations-Katalysators oxidiert, so ist 6-Hydroperoxycapronsäure nur in geringen Mengen enthalten.

Wurde die Cyclohexan-Oxidation ohne Katalysator durchgeführt, so muss die entstandene 6-Hydroperoxycapronsäure ebenso wie 5-Formylvaleriansäure zu 6-Hydroxycapronsäure hydriert werden. Diese Hydrierung findet vor dem Schritt c) des erfindungsgemäßen Verfahrens statt.

Da bei der Hydrierung, die gegebenenfalls vor dem Schritt c) des erfindungsgemäßen Verfahrens stattfindet, im einen Fall Hydroperoxy-, im anderen Fall eine Aldehydgruppe hydriert werden muss, unterscheiden sich die optimalen Hydrierbedingungen beider Verbindungen.

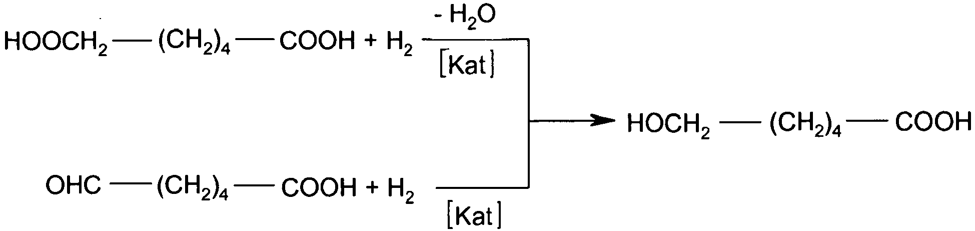

Da die Hydroperoxycapronsäure auch rein thermisch, aber weniger selektiv als bei einer Hydrierung, in 6-Hydroxycapronsäure übergehen kann, wird sie gemäß DE-A 1 951 250 in Gegenwart von Palladium, Rhodium- oder Platin-Katalysatoren bei 15 bis 130°C, vorzugsweise 50 bis 100°C, also bei moderaten Temperaturen hydriert.

Keto- und Aldehydgruppen werden unter den Bedingungen der 6-Hydroperoxycapronsäure-Hydrierung in DE-A 1 951 250 nicht hydriert. Hierfür sind höhere Temperaturen und Drücke notwendig.

Die Hydrierung der DCL, die gegebenenfalls vor Schritt c) des erfindungsgemäßen Verfahrens durchgeführt wird, kann in einem Reaktor oder in zwei hintereinander geschalteten Reaktoren durchgeführt werden. Verwendet man zwei Reaktoren, so können die beiden Reaktoren den gleichen Katalysator oder zwei verschiedene Katalysatoren enthalten. Dabei können sich die beiden Reaktoren in der Hydriertemperatur und dem Wasserstoffpartialdruck unterscheiden.

Es ist weiterhin möglich, die Hydrierung, die gegebenenfalls vor Schritt c) des erfindungsgemäßen Verfahrens durchgeführt wird, in einem Reaktor, der nur mit einem Katalysator gefüllt ist, so durchzuführen, dass die Hydriertemperatur im Reaktor innerhalb eines gewünschten Temperaturbereichs ansteigt.

Die Hydrierung, die gegebenenfalls vor Schritt c) des erfindungsgemäßen Verfahrens durchgeführt wird, erfolgt bei 10 bis 200°C, bevorzugt 30 bis 180°C, besonders bevorzugt 50 bis 170°C. Der Wasserstoff-Partialdruck beträgt dabei 1 bis 100 bar, bevorzugt 10 bis 80 bar, besonders bevorzugt 30 bis 60 bar.

Für die katalytische Hydrierung, die gegebenenfalls vor Schritt c) des erfindungsgemäßen Verfahrens durchgeführt wird, dienen Katalysatoren, die mindestens ein Metall der 7. bis 12. Gruppe des Periodensystems, beispielsweise Ruthenium, Palladium, Rhodium, Nickel, Cobalt, Eisen, Rhenium, Platin, Iridium, Kupfer, Osmium und Zink enthalten.

Gut geeignet sind weiterhin sogenannte Vollkatalysatoren, die keine Träger enthalten und aus Metallen, Metalloxiden oder deren Gemischen bestehen. Bevorzugt sind dabei Eisen- und insbesondere Cobalt-Vollkatalysatoren.

Bevorzugt sind dabei die Metalle Palladium, Ruthenium, Nickel, Cobalt, Rhenium und Kupfer. Diese Metalle können sowohl in Form der Metalle als auch deren Verbindungen, wie z. B. Oxide und Sulfide eingesetzt werden.

Die Metalle oder Metallverbindungen können ohne Träger verwendet werden. Bevorzugt werden sie jedoch auf Träger, wie z. B. TiO₂, Al₂O₃, ZrO₂, SiO₂, HfO₂, Kohle, Zeolithe oder deren Gemische aufgebracht. Diese Trägerkatalysatoren können in verschiedensten Konfektionierungsformen, wie z. B. Strängen, Tabletten oder Ringen verwendet werden.

Kupfer, Nickel- und Cobalt können bevorzugt in Form von Raney-Nickel, Raney-Kupfer oder Raney-Cobalt eingesetzt werden. Auch die Raney-Katalysatoren können in allen bekannten Konfektionierungsformen, z. B. als Tabletten, Stränge oder Granulate, eingesetzt werden. Geeignete Raney-Kupfer-Katalysatoren sind z. B. Raneykupfer-Nuggets, die in WO 99/03.801 beschrieben sind.

Besonders geeignet für die Hydrierung, die gegebenenfalls vor Schritt c) des erfindungsgemäßen Verfahrens durchgeführt wird, ist weiterhin ein Katalysator, enthaltend auf Titandioxid-Formkörpern geträgertes Ruthenium, wobei die Titandioxid-Formkörper durch Behandeln von marktüblichem Titandioxid vor oder nach dem Formen mit 0,1 bis 30 Gew.-% einer Säure, in der Titandioxid schwer löslich ist, erhalten werden, der in dem erfindungsgemäßen Verfahren verwendet wird. Ruthenium kann dabei sowohl in Form des reinen Metalls als auch als dessen Verbindung, beispielsweise Oxyd oder Sulfid, eingesetzt werden.

Das katalytisch aktive Ruthenium wird nach an sich bekannten Verfahren, bevorzugt auf vorgefertigtes TiO₂ als Trägermaterial aufgebracht.

Ein für die Verwendung in dem Ruthenium enthaltenden Katalysator bevorzugt geeigneter Titandioxid-Träger kann entsprechend DE 197 38 464 durch Behandeln von marktüblichem Titandioxid vor oder nach dem Formen mit 0,1 bis 30 Gew.-% einer Säure, bezogen auf Titandioxid, in der das Titandioxid schwer löslich ist, erhalten werden. Bevorzugt wird Titandioxid in der Anatas-Modifikation verwendet. Als derartige Säuren sind beispielsweise Ameisensäure, Phosphorsäure, Salpetersäure, Essigsäure oder Stearinsäure geeignet.

Die Aktivkomponente Ruthenium kann in Form einer Rutheniumsalzlösung auf den so erhaltenen Titandioxidträger in einer oder mehreren Tränkstufen aufgebracht werden. Anschließend wird der getränkte Träger getrocknet und gegebenenfalls calciniert. Es ist jedoch auch möglich Ruthenium aus einer Rutheniumsalzlösung, bevorzugt mit Natriumcarbonat, auf ein als Pulver in wässriger Suspension vorliegendes Titandioxids zu fällen. Die ausgefällten Niederschläge werden gewaschen, getrocknet, gegebenenfalls calciniert und verformt. Weiterhin können flüchtige Rutheniumverbindungen, wie beispielsweise Rutheniumacetylacetonat oder Rutheniumcarbonyl, in die Gasphase überführt werden und in an sich bekannter Weise auf den Träger aufgebracht werden (Chemical vapor deposition).

Die so erhaltenen, geträgerten Katalysatoren können in allen bekannten Konfektionierungsformen vorliegen. Beispiele sind Stränge, Tabletten oder Granulate. Vor ihrer Verwendung werden die Rutheniumkatalysatorvorläufer durch Behandlung mit wasserstoffhaltigem Gas, bevorzugt bei Temperaturen über 100 °C reduziert. Bevorzugt werden die Katalysatoren vor ihrem Einsatz im erfindungsgemäßen Verfahren bei Temperaturen von 0 bis 50 °C, bevorzugt bei Raumtemperatur, mit sauerstoffhaltigen Gasgemischen, bevorzugt mit Luft-Stickstoffgemischen, passiviert. Es ist auch möglich, den Katalysator in oxidischer Form in den Hydrierreaktor einzubauen und unter Reaktionsbedingungen zu reduzieren.

Der erfindungsgemäß besonders bevorzugte Katalysator weist einen Rutheniumgehalt von 0,1 bis 10 Gew.-%, bevorzugt von 2 bis 6, bezogen auch das Gesamtgewicht des Katalysators aus katalytisch aktivem Metall und Träger, auf. Der erfindungsgemäße Katalysator kann einen Schwefelgehalt von 0,01 bis 1 Gew.-%, bezogen auf das Gesamtgewicht des Katalysators, aufweisen (Schwefel-Bestimmung: coulometrisch).

Die Rutheniumoberfläche beträgt dabei von 1 bis 20 m²/g, bevorzugt von 5 bis 15 und die BET-Oberfläche (bestimmt nach DIN 66 131) von 5 bis 500 m²/g, bevorzugt von 50 bis 200 m²/g.

Die gegebenenfalls in der Hydrierung vor Schritt c) des erfindungsgemäßen Verfahrens eingesetzten Katalysatoren weisen ein Porenvolumen von 0,1 bis 1 ml/g auf. Weiterhin zeichnen sich die Katalysatoren durch eine Schneidhärte von 1 bis 100 N aus.

Die Hydrierkatalysatoren, die gegebenenfalls vor Schritt c) des erfindungsgemäßen Verfahrens verwendet werden, können im Reaktionsgemisch suspendiert sein. Bevorzugt ist, sie im Hydrierreaktor fest anzuordnen. Die Hydrierung kann diskontinuierlich oder bevorzugt kontinuierlich durchgeführt werden. Das Reaktionsgemisch kann dabei in Sumpf- oder Rieselfahrweise über den Katalysator geleitet werden.

Für die Veresterung gemäß Schritt c) des erfindungsgemäßen Verfahrens der in der DCL enthaltenen Carbonsäuren sind Polyole, Diole, besonders aber alpha, omega-Diole mit zwei bis zwölf Kohlenstoffatomen oder Gemische dieser Diole geeignet. Beispiele für derartige mehrwertige Alkohole sind Glycerin, Trimethylolpropan, Propylenglykol, Ethylenglykol, 1,3-Propandiol, 1,4-Butandiol, 1,5-Pentandiol, 1,6-Hexandiol, 1,2-Cyclohexandiol, 1,4-Cyclohexandiol, 1,8-Octandiol, 1,10-Decandiol, 1,12-Dodecandiol oder Gemische dieser Diole. Bevorzugt sind 1,4-Butandiol, 1,5-Pentandiol, 1,6-Hexandiol, 1,2-Cyclohexandiol, 1,4-Cyclohexandiol oder Gemische dieser Diole. Besonders bevorzugt ist 1,6-Hexandiol, da dieser Veresterungsalkohol dem Zielprodukt der Hydrierung entspricht.

Bevorzugt ist weiterhin, anstelle von reinem 1,6-Hexandiol einen Teil des Hydrieraustrags für die Veresterung der DCL gemäß Schritt c) des erfindungsgemäßen Verfahrens, die Hauptmenge für die destillative Aufarbeitung zu 1,6-Hexandiol zu verwenden. Das Gewichtsverhältnis von Hydrieraustrag für die Veresterung zu Hydrieraustrag für die 1,6-Hexandiolgewinnung beträgt dabei von 2,5:1 bis 0,8:1, bevorzugt von 1,5:1 bis 0,9:1. Der Hydrieraustrag enthält im allgemeinen 60 bis 90 Gew.-% 1,6-Hexandiol, 2 bis 10 Gew.-% 1,5-Pentandiol, 1,4-Butandiol, 1,2-Cyclohexandiol und 1,4-Cyclohexandiol (jeweils weniger als 5 Gew.-%), weiterhin bis 5 Gew.-% Monoalkohole wie z.B. n-Butanol, n-Pentanol und n-Hexanol und 1 bis 10 Gew.-% oligomere oder polymere Hochsieder gegenüber 1,6-Hexandiol.

Besonders bevorzugt ist, einen Teil des Hydrieraustrags nach destillativer Abtrennung von Leichtsiedern und Hochsiedern für die Veresterung der DCL in Schritt c) des erfindungsgemäßen Verfahrens zu verwenden. Unter Leicht- und Hochsiedern sind dabei Verbindungen zu verstehen, die niedriger bzw. höher als die im Hydrieraustrag enthaltenen Diole sieden.

Der DCL wird zur Veresterung in Schritt c) des erfindungsgemäßen Verfahrens, ein Diol, insbesondere ein alpha, omega-Diol wie 1,6-Hexandiol oder ein Gemisch aus Diolen hinzugefügt. Als Diolgemisch kann eine Teilmenge des Hydrieraustrags oder eine Teilmenge des Hydrieraustrags nach Abtrennung von Hoch- und Leichtsiedern dienen. Das Massenverhältnis von DCL zu Diolen beträgt dabei 1 zu 0,2 bis 1 zu 0,8, bevorzugt 1 zu 0,3 bis 1 zu 0,7, besonders bevorzugt 1 zu 0,4 bis 1 zu 0,6.

Wasserabtrennung gemäß Schritt b) und Veresterung gemäß Schritt c) des erfindungsgemäßen Verfahrens werden bevorzugt in einem Verfahrensschritt durchgeführt. Hierzu können Rührreaktoren, Strömungsrohre und/oder Kolonnen verwendet werden. Bevorzugt erfolgen Wasserabtrennung und Veresterung in mindestens einem Reaktor mit aufgesetzter Destillationskolonne. Um einen vollständigen Umsatz bei der Veresterung der Carbonsäuren und eine vollständige Wasserabtrennung zu erreichen, arbeitet man mit 2 bis 6, bevorzugt mit 3 bis 5 hintereinander geschalteten Reaktoren mit aufgesetzter Kolonne.

Die Veresterungsreaktion zu den Oligo- und/oder Polyestern der DCL sowie gemäß Schritt c) des erfindungsgemäßen Verfahrens kann ohne Zusatz eines Katalysators ablaufen. Es kann aber auch zur Erhöhung der Reaktionsgeschwindigkeit ein Katalysator für die Veresterung zugesetzt werden. Dabei kann es sich um einen homogenen, gelösten oder um einen heterogenen Katalysator handeln.

Als homogene Katalysatoren für die Veresterung seien beispielhaft Schwefelsäure, Phosphorsäure, Salzsäure, Sulfonsäuren wie p-Toluolsulfonsäure, Heteropolysäuren wie Wolframatophosphorsäure oder Lewissäuren wie z. B. Aluminium-, Vanadium-, Titan-, Bor-Verbindungen genannt. Bevorzugt sind Mineralsäuren, insbesondere Schwefelsäure. Das Gewichtsverhältnis von homogenem Katalysator zu Carbonsäure beträgt in der Regel 0,0001 bis 0,5, bevorzugt 0,001 bis 0,3.

Als heterogene Katalysatoren sind saure oder supersaure Materialien, z.B. saure und supersaure Metalloxide wie SiO₂, Al₂O₃, SnO₂, ZrO₂, Schichtsilikate oder Zeolithe, die alle zur Säureverstärkung mit Mineralsäureresten wie Sulfat oder Phosphat dotiert sein können, oder organische Ionentauscher mit Sulfonsäure-, oder Carbonsäuregruppen geeignet. Die festen Katalysatoren können als Festbett angeordnet oder als Suspension eingesetzt werden.

Bevorzugt wird ohne Katalysator verestert.

Die Sumpftemperatur in den Reaktoren mit aufgesetzter Kolonne beträgt 200 bis 250°C. Die Veresterung und Wasserabtrennung kann bei Drücken von 0,1 bis 5 bar, bevorzugt 0,5 bis 3 bar, besonders bevorzugt bei 1 bar durchgeführt werden. Die Verweilzeit, gerechnet über alle Rührkessel beträgt 0,5 bis 12 Stunden, bevorzugt 1 bis 11 Stunden, besonders bevorzugt 2 bis 10 Stunden.

Als Kopfprodukt der aufgesetzten Kolonnen wird das in der DCL enthaltene und das bei der Veresterung entstandene Wasser erhalten. Das Kopfprodukt kann weiterhin organische Nebenprodukte wie z. B. niedere Monocarbonsäuren, z. B. Ameisensäure, enthalten.

Als Sumpfprodukt des letzten Reaktors fällt ein Gemisch aus Oligo- und Polyestern an, das sich aus den in der DCL enthaltenen Carbonsäuren, den Cyclodiolen und den zugesetzten Diolen bildete. Im Sumpfprodukt sind außerdem nicht umgesetzte Diole enthalten. Dieses Gemisch wird für die anschließende katalytische Hydrierung in Schritt d) des erfindungsgemäßen Verfahrens verwendet.

Die Vollständigkeit des Umsatzes der in dem Carbonsäuregemisch vorhandenen freien Carboxylgruppen wird mit der nach der Veresterung gemessenen Säurezahl (mg KOH/g) festgestellt. Sie beträgt abzüglich der gegebenenfalls zugesetzten Säure als Katalysator 1 bis 20, bevorzugt 2 bis 15, besonders bevorzugt 5 bis 10 mg KOH/g.

Wurde zur Veresterung eine gelöste Säure als Katalysator eingesetzt, wird das Estergemisch zweckmäßig mit einer Base neutralisiert, wobei pro Säureäquivalent des Katalysators 1 bis 1,5 Basenäquivalente zugesetzt werden. Als Basen werden in der Regel Alkali- oder Erdalkalimetalloxide, -Carbonate, -Hydroxyde oder -Alkoholate, oder Amine in Substanz oder in dem Veresterungsalkohol gelöst verwendet.

Die Hydrierung des Oligo- und/oder Polyester-Gemischs sowie die Hydrierung in Schritt d) des erfindungsgemäßen Verfahrens erfolgt katalytisch in der Flüssigphase in Gegenwart von fest angeordneten oder suspendierten, bevorzugt fest angeordneten Katalysatoren. Gearbeitet wird bei Temperaturen zwischen 100 und 350°C, bevorzugt 120 und 300°C, besonders bevorzugt 140°C und 280°C und Drucken von 30 bis 350 bar, bevorzugt 40 bis 320 bar, besonders bevorzugt 50 bis 300 bar. Die Katalysator-Belastung beträgt 0,2 bis 1,5 kg Oligoester/kg Katalysator x h.

Die Hydrierung des Oligo- und/oder Polyester-Gemischs sowie die Hydrierung in Schritt d) des erfindungsgemäßen Verfahrens kann grundsätzlich in nur einem Reaktor durchgeführt werden. Diese Fahrweise besitzt jedoch Nachteile: Esterhydrierungen sind stark exotherm und müssen dazu noch bei hohen Temperaturen durchgeführt werden. So beträgt die Hydriertemperatur nach US-A 3,524,892, wo die Hydrierung von aus DCL hergestellten Oligoestern in Gegenwart von durch Bariumoxid dotiertem Kupferchromit erfolgte, 260 bis 270 °C. Für die sichere Wärmeabfuhr aus dem Reaktor muss ein hoher Aufwand getrieben werden.

Daher wird die Hydrierung des Oligo- und/oder Polyester-Gemischs sowie die Hydrierung in Schritt d) des erfindungsgemäßen Verfahrens bevorzugt in mindestens zwei hintereinander geschalteten Reaktoren durchgeführt. Arbeitet man mit Festbettkatalysatoren, so kann man den Hydrierfeed in Sumpf- oder Rieselfahrweise über den Katalysator leiten. Beim Arbeiten in Sumpffahrweise wird Wasserstoffgas in den mit der flüssigen Reaktionsmischung gefluteten Reaktor eingeleitet, wobei der Wasserstoff das Katalysatorbett in aufsteigenden Gasperlen passiert. Beim Arbeiten in Rieselfahrweise lässt man das flüssige Estergemisch in dem Reaktor, der unter Wasserstoffdruck steht, über das in diesem angeordnete Katalysatorbett rieseln, wobei sich auf dem Katalysator ein dünner Flüssigkeitsfilm ausbildet.

Nach einer besonders bevorzugten Ausführungsform verwendet man mehrere Reaktoren, wobei im ersten Reaktor der überwiegende Teil der Ester, bis zu einem Umsatz von 80 bis 98%, bevorzugt von 90 bis 95 % hydriert wird. Der erste Reaktor wird bevorzugt mit Flüssigkeitskreislauf zur Wärmeabfuhr durch Wärmetauscher und der nachfolgende oder die nachfolgenden Reaktoren bevorzugt im geraden Durchgang, ohne Umlauf zur Vervollständigung des Umsatzes betrieben. Diese Fahrweise wird als Kreislauffahrweise bezeichnet.

Die Hydrierung des Oligo- und/oder Polyester-Gemischs sowie die Hydrierung in Schritt d) des erfindungsgemäßen Verfahrens kann diskontinuierlich, bevorzugt kontinuierlich erfolgen.

Die Hydrierung des Oligo- und/oder Polyester-Gemischs sowie die Hydrierung in Schritt d) des erfindungsgemäßen Verfahrens wird im allgemeinen mit dem bei der Veresterung anfallenden, überschüssige Diole enthaltenden Estergemisch ohne zusätzliches Lösungsmittel durchgeführt. Es kann aber auch vorteilhaft sein, in Gegenwart eines unter den Reaktionsbedingungen inerten Lösungsmittels zu arbeiten. Als Lösungsmittel kommen z. B. alle für die Veresterung verwendeten Diole, weiterhin Tetrahydrofuran, Dioxan und Monoalkohole mit 1 bis 6 Kohlenstoffatomen, wie z.B. Methanol, Ethanol, Propanole, n-Butanol, n-Hexanol oder Gemische der genannten Verbindungen in Frage. Die Lösungsmittel-Menge beträgt dabei 5 bis 50 Gew.-%, bevorzugt 10 bis 30 Gew.-%, bezogen auf das Estergemisch.

Bevorzugt wird die Hydrierung des Oligo- und/oder Polyester-Gemischs sowie die Hydrierung in Schritt d) des erfindungsgemäßen Verfahrens ohne Lösungsmittel durchgeführt.

Erfindungsgemäß wird dem bei der Veresterung anfallenden Estergemisch eine Base zu zudosieren, ausgewählt aus der Gruppe bestehend aus Lithium-, Natrium- und Kalium-Alkoholate, besonders bevorzugt Natriummethylat. Die Menge an Base beträgt dabei 20 bis 180 ppm, bevorzugt 30 bis 90 ppm, bezogen auf das Estergemisch. Bei einem Estergemisch mit Restsäurezahl > 1 mg KOH / g, werden die Restsäuren nur in unbedeutsamen Mengen neutralisiert. Die zugegebene Base dient dazu, die Bildung von Nebenprodukten, die sonst bei der Hydrierung entstehen könnten, wie z.B. Hexanol oder Etherverbindungen, zu unterdrücken.

Die Hydrierung des Oligo- und/oder Polyester-Gemischs sowie die Hydrierung in Schritt d) des erfindungsgemäßen Verfahrens erfolgt in Gegenwart des Katalysator-Formkörpervorläufers der neben Kupferoxid, Aluminiumoxid und mindestens einem der Oxide des Lanthans, Wolframs, Molybdäns, Titans, Zirkoniums oder Eisens auch noch metallisches Kupfer, Kupferblättchen, pulverförmigen Zement, Graphit oder ein Gemisch enthält wie er bereits im Verfahren zur Hydrierung der Oligo- und Polyester der DCL mit Diolen beschrieben wurde. Der Katalysator und seine Herstellung sind in WO 2004/085356, WO 2006/005505 und WO 2007/006719 beschrieben.

Die Formkörper werden vor dem Einsatz als Katalysator in an sich bekannter Weise durch Behandlung mit reduzierenden Medien aktiviert. Das Aktivieren erfolgt entweder vorab in einem Reduktionsofen oder nach dem Einbau im Reaktor. Ist der Katalysator-vorläufer vorab im Reduktionsofen aktiviert worden, wird er in den Reaktor eingebaut und direkt unter Wasserstoffdruck mit der Hydrierlösung beschickt.

Bei der Hydrierung des Oligo- und/oder Polyester-Gemischs sowie der Hydrierung in Schritt d) des erfindungsgemäßen Verfahrens wird ein hoher Oligo- und Polyesterumsatz erzielt. Der Umsatz beträgt in Gegenwart der erfindungsgemäßen Katalysatoren mehr als 90 %, bevorzugt mehr als 95 %, besonders bevorzugt mehr als 99 %. Nicht umgesetzte Oligo- und Polyester stellen gegenüber 1,6-Hexandiol Hochsieder dar und fallen beim Destillieren als Sumpfprodukte an.

Bevorzugt wird ein Teil des Hydrieraustrags gemäß dem Schritt e) des erfindungsgemäßen Verfahrens, der ein Gemisch aus Diolen darstellt, anstelle von reinem 1,6-Hexandiol für die Veresterung der DCL verwendet. Der Vorteil dieses Verfahrens besteht darin, dass andere Diole, wie 1,5-Pentandiol, 1,4-Butandiol, 1,4-Cyclohexandiol, 1,2-Cyclohexandiol, die zum Teil Nebenprodukte darstellen, das 1,6-Hexandiol ersetzen. Dadurch werden Verluste an 1,6-Hexandiol, das Nebenreaktionen eingeht, wesentlich vermindert.

Der Hydrieraustrag wird in einer ersten Kolonne von Hoch- und Leichtsiedern gegenüber den Diolen 1,6-Hexandiol, 1,5-Pentandiol, 1,4-Butandiol, 1,4-Cyclohexandiol, 1,2-Cyclohexandiol befreit. Die Kolonne besitzt 1 bis 30 theoretische Böden. Gearbeitet wird bei Sumpftemperaturen von 120 bis 250 °C und Drücken von 5 bis 500 mbar.

Das Kopfprodukt enthält 75 bis 95 Gew.-% 1,6-Hexandiol, 3 bis 10 Gew.-% 1,5-Pentandiol, 1,4-Butandiol, 1,2-Cyclohexandiol und 1,4-Cyclohexandiol (jeweils weniger als 5 Gew.-%), weiterhin bis 5 Gew.-% Monoalkohole wie z. B. n-Butanol, n-Pentanol und n-Hexanol und weniger als 5 Gew.-% höher siedende Komponenten gegenüber 1,6-HDO.
Im Sumpfprodukt sind nicht umgesetzte Oligo- und Polyester, enthalten. Da der Oligo-und Polyester-Umsatz bis zu ≥ 97 % beträgt, enthält das Sumpfprodukt ≤ 3 % zu 1,6-Hexandiol hydrierbare Produkte. Es kann daher verworfen werden.

Das gewonnene Destillat wird in eine zweite Kolonne zugeleitet, in der die Feinreinigung des 1,6-Hexandiols erfolgt. Dabei werden 1,6-Hexandiol Reinheiten von > 97 % erzielt.

In einer besonders bevorzugten Ausführungsform der Veresterung gemäß Schritt c) des erfindungsgemäßen Verfahrens wird ein Teil des von Hoch- und Leichtsiedern befreiten Hydrieraustrags in die Veresterung in Schritt c) zurückgeführt. Diese Variante besitzt den zusätzlichen Vorteil, dass die Produktströme im Verfahren verkleinert werden.

Das erfindungsgemäße Verfahren erlaubt somit in wirtschaftlicher Weise, aus einem Abfallprodukt reines 1,6-Hexandiol zu gewinnen.

Es war nicht vorauszusehen, dass der erfindungsgemäße Katalysator bei Verwendung von Oligoestern als Feed und Restsäurezahlen von 1 bis 20 über lange Reaktionszeiten (Beispiel: 900 Stunden) eine hohe Aktivität bei hoher Seitendruckfestigkeit und damit hoher mechanischer Festigkeit beibehalten würde.

Hinzu kommt, dass man erfindungsgemäß mit ≥ 95 % deutlich höhere 1,6-Hexandiol-Ausbeuten erzielt als nach dem Stand der Technik.

### Ausführungsbeispiel

Herstellung von 1,6-Hexandiol (1,6-HDO) aus einer Dicarbonsäurelösung (DCL) durch Veresterung mit einem Diol-Gemisch, anschließende Hydrierung und Aufarbeitung

### 1. Abmischung eines Dicarbonsäure-Rohhexandiolgemischs

Die verwendete Dicarbonsäure-Lösung wurde durch Extraktion eines Reaktionsaustrags, der aus der Oxidation von Cyclohexan mit Luft stammte, mit Wasser erhalten.

Das Roh-1,6-Hexandiol-Gemisch wurde durch destillative Abtrennung von Hoch- und Leichtsiedern (gegenüber den Diolen) aus dem Hydrieraustrag der Oligo- und Polyester-Hydrierung hergestellt (siehe 4a/b.).

Zu 750 kg einer DCL (Säure-Zahl: 267 mg KOH/g), enthaltend u. a. Adipinsäure (ADS, 21 Gew. %), 6-Hydroxycapronsäure (HCS, 18 Gew. %) und Wasser (45 Gew. %), anteilig in Form von Oligomeren, wurden 337 kg eines Roh-1,6-Hexandiol-Gemischs zugegeben. Die Roh-1,6-HDO-Charge enthält u.a. 1,6-Hexandiol (ca. 80 Gew.%), 1,5-Pentandiol, 1,4-Butandiol, 1,4-Cyclohexandiol und 1,2-Cyclohexandiol. Die entstandene wässrige Lösung (DCL-HDO-Gemisch) enthielt 1,6-HDO (23 Gew.%), ADS (15 Gew.%) und HCS (12,0 Gew.%) als Hauptkomponenten (anteilig im Form von Oligomeren).

### 2. Herstellung eines oligomeren Estergemisches

Das DCL-HDO-Gemisch der Stufe 1 wurde kontinuierlich in einen Verdampfer (Entwässerungsstufe, 150 °C, Umgebungsdruck) bei einem Durchsatz von 260 g/h dosiert. Dabei wurden Wasser und leichtsiedende Komponenten abdestilliert (98 g/h). Der Sumpfaustrag wurde anschließend in eine 5-stufige Rührkesselkaskade überführt (174 g/h, 220 °C, 1-1,4 bar abs.), in der die Veresterung bis zum fast vollständigen Umsatz gebracht wurde (SZ < 10 mg KOH/g, 98% Umsatz). In der Esterkaskade wurden ebenfalls leichtsiedende Komponenten abdestilliert (12 g/h), die in die Entwässerungsstufe rückgeführt wurden. Man erhielt als Sumpfaustrag ein oligomeres Gemisch enthaltend hauptsächlich Ester aus den ursprünglich zugeführten Carbonsäurederivaten und Diolen (162 g/h, 62 % Gewichtsausbeute, bezogen auf den gesamten Zulauf).

### 3. Hydrierung des oligomeren Ester-Gemisches

Die oligomeren Ester der Stufe 2 wurden mit 60 ppm Natriummethylat versetzt und anschließend kontinuierlich an einem Cu-Katalysator hydriert. Der Katalysator wurde nach WO 2007/6719 Beispiel 3 hergestellt und aktiviert.

Das Reaktorsystem bestand aus einem Hauptreaktor (Rohrreaktor, 400 mL, 600 g Katalysator) und einem Nachreaktor (Rohrreaktor, 100 mL, 150 g Katalysator). Der Hydrierzulauf wurde in Rieselfahrweise über den fest angeordneten Katalysator geleitet. Um die sich bei der Hydrierung entwickelnde Wärme abzuführen, wurde der Hauptreaktor mit Flüssigkeitskreislauf, der Nachreaktor im geraden Durchgang betrieben. Der Hydrierreaktor wurde 900 h bei 240 °C / 255 bar H₂ betrieben. Bei einem Zulauf von 240 g/h (Katalysatorbelastung = 0,60 kgL⁻¹h⁻¹, Hauptreaktor) wurde ein Umsatz von 98 % erzielt. Der Hydrieraustrag wurde anschließend in einem Behälter auf Umgebungsdruck entspannt und auf Umgebungstemperatur gekühlt. Man erhielt Austräge, deren Gehalt an 1,6-Hexandiol 72 Gew. % betrug. Die Hydrierung verlief mit > 95 % Ausbeute zu 1,6-HDO (die Ausbeute bezieht sich auf die in der DCL vorhandenen C6-Komponenten, die durch Hydrierung zu 1,6-HDO führen können: 6-Hydroxycapronsäure, 6-Oxocapronsäure (5-Formylvaleriansäure), Adipinsäure und Dihydromuconsäure). Der Katalysator wurde ausgebaut und anschließend analysiert. Der Ausbaukatalysator wies eine Seitendruckfestigkeit von 31 N (Originalkatalysator: 48 N) auf.

Der Gehalt an 1,6-Hexandiol wurde per GC ermittelt: DB-5 (Agilent J&W), 30 m x 0,2 mm x 1 µm; Temperaturprofil: 60°C (5 min) → 220°C (16°C/min, 10 min) → 260°C (20°C/min, 21 min) → 290°C (20°C/min, 10 min). Diethylenglykol Dimethylether (DEGDME) wurde als interner Standard verwendet, t_{R}(DEGDME) = 8,8 min, t_{R}(1.6-HDO) = 11,8 min.

### 4 a Abtrennung von Hoch- und Leichtsiedern aus dem Hydrieraustrag

In einer Destillationsblase mit aufgesetzter Kolonne (DN50, Einbauten 1 m, Gewebepackung 750 m²/m³) wurden Hydrierausträge (917 g) der Stufe 3 destillativ aufgetrennt. Bei 1 bar und 150 °C Sumpftemperatur wurden Leichtsieder (25 g) abgezogen. Anschließend wurde der Druck auf 150 mbar reduziert und die Sumpftemperatur auf bis zu 225 °C erhöht. Das gewonnene Destillat wurde zu 30 % zur Kolonne am Kopf rückgeführt, der überwiegende Teil wurde gesammelt. Es fielen 840 g Destillat an (enthielt u. a. 79 Gew.-% 1,6-HDO, 9 Gew.-% 1,5-PDO). Die Hochsieder reicherten sich im Sumpf an (41 g, 4,5 Gew.-%). Ein Teil der gesammelten Destillate wird als Roh-1,6-HDO-Charge mit der DCL abgemischt (siehe Stufe 1), wobei der andere Teil in die Reindestillation überführt wird.

### 4 b Reindestillation

Eine Roh-1,6-HDO-Charge aus der Hochsiederabtrennung wurde bei 50 mbar fraktioniert aufdestilliert (Rücklaufverhältnis von 10 : 1, Kopf-Abzug von ca. 50 g/h). Nach Abtrennung von anderen leichtsiedenden Diolen (u. a. 1.5-Pentandiol) konnte 1,6-HDO mit einer Reinheit von > 97 % erhalten werden.

## Patentansprüche

1. Verfahren zur Hydrierung von Oligo- und/oder Polyestern, erhältlich durch Veresterung einer Dicarbonsäurelösung mit einem Diol oder Diolgemisch, wobei dem anfallenden Estergemisch eine Base, ausgewählt aus der Gruppe bestehend aus Lithium-, Kalium- und Natrium-Alkoholat, zudosiert wird, und wobei die Hydrierung mittels eines Katalysator-Formkörpers, dessen Vorläufer gemäß einem Verfahren herstellbar ist, in dem
(i) ein oxidisches Material, umfassend Kupferoxid, Aluminiumoxid und mindestens eins der Oxide des Lanthans, Wolframs, Molybdäns, Titans, Zirkoniums oder Eisen bereitgestellt wird,
(ii) dem oxidischen Material pulverförmiges metallisches Kupfer, Kupferblättchen, pulverförmiger Zement, Graphit oder ein Gemisch davon zugegeben wird
(iii) das aus (ii) resultierende Gemisch zu einem Formkörper verformt wird,
durchgeführt wird, wobei das oxidische Material erhältlich ist durch simultanes oder nacheinander Fällen der Aktivkomponente Kupfer, der Komponente Aluminium und der Komponente mindestens eines der Oxide des Lanthans, Wolframs, Molybdäns, Titans oder Zirkoniums und anschließendes Trocknen und Calcinieren und nach der Verformung nach Schritt (iii) der Katalysator-Formkörper nochmals calciniert wird.

2. Verfahren nach Anspruch 1, **dadurch gekennzeichnet, dass** das oxidische Material
(a) Kupferoxid mit einem Anteil im Bereich von 50 ≤ x ≤ 80, vorzugsweise 55 ≤ x ≤ 75 Gew.-%,
(b) Aluminiumoxid mit einem Anteil im Bereich von 15 ≤ y ≤ 35, vorzugsweise 20 ≤ y ≤ 30 Gew.% und
(c) mindestens eins der Oxide des Larithans, Wolframs, Molybdäns, Titans oder Zirkoniums mit einem Anteil im Bereich von 2 ≤ z s 20, bevorzugt 3 ≤ z ≤ 15 Gew.-%,
jeweils bezogen auf das Gesamtgewicht des oxidischen Materials nach Calcinierung, wobei gilt: 80 ≤ x + y + z ≤ 100, insbesondere 95 ≤ x + y + z ≤ 100, wobei Zement nicht dem oxidischen Material im obigen Sinne zugerechnet wird, umfasst.

3. Verfahren nach einem der Ansprüche 1 oder 2, **dadurch gekennzeichnet, dass** durch die Zugabe das pulverförmige metallische Kupfer, die Kupferblättchen, der pulverförmige Zement oder Graphit oder das Gemisch davon in einem Anteil im Bereich von 0,5 bis 40 Gew.-%, bezogen auf das Gesamtgewicht des oxidischen Materials nach erster Calcinierung beträgt.

4. Verfahren nach einem der Ansprüche 1 bis 3, **dadurch gekennzeichnet, dass** zur Veresterung verwendete Diole alpha, omega-Diole mit zwei bis zwölf Kohlenstoffatomen oder Gemische dieser Diole sind.

5. Verfahren nach einem der Ansprüche 1 bis 4, **dadurch gekennzeichnet, dass** der aus Schritt (iii) erhaltende Formkörper noch mit siedendem Wasser und/oder Dampf behandelt wird.

6. Verfahren zur Herstellung von 1,6-Hexandiol umfassend folgende Schritte
a) Oxidation von Cyclohexan mit Sauerstoff oder Sauerstoff enthaltenden Gasen zu Gemischen aus Cyclohexanol, Cyclohexanon und Carbonsäuren mit bis zu sechs Kohlenstoffatomen,
b) Umsatz des nach a) erhaltene Reaktionsgemisches mit Wasser und Abtrennung der Dicarbonsäurelösung aus dem flüssigen zweiphasigen Reaktionsgemisch,
c) Veresterung der aus b) erhaltenen Dicarbonsäurelösung mit einem Alkohol,
d) katalytische Hydrierung des aus c) erhaltenen Estergemisches und
e) Destillation des aus d) erhaltenen Hydrieraustrags,
**dadurch gekennzeichnet, dass** man
die Veresterung in c) mit mindestens einem Diol mit zwei bis zwölf Kohlenstoffatomen durchführt,
dem in c) anfallenden Estergemisch eine Base, ausgewählt aus der Gruppe bestehend aus Lithium-, Kalium- und Natrium-Alkoholat, zudosiert, und
das in c) erhaltene Veresterungsgemisch in der Flüssigphase in Gegenwart eines Katalysator-Formkörpers hydriert, dessen Vorläufer erhältlich ist durch
(i) Bereitstellung eines oxidischen Material nach erster Calcinierung enthaltend Kupferoxid, Aluminiumoxid und mindestens eins der Oxide des Lanthans, Wolframs, Molybdäns, Titans, Zirkoniums oder Eisen,
(ii) Zugabe von pulverförmigem metallischem Kupfer, Kupferblättchen, pulverförmigem Zement, Graphit oder einem Gemisch zu dem oxidischen Material in aus Schritt i)
(iii) Formung des aus (ii) resultierenden Gemisches zu einem Formkörper und
(iv) zweiter Calcinierung des aus Schritt (iii) erhaltenen Formkörpers.

7. Verfahren nach Anspruch 6, **dadurch gekennzeichnet, dass** die in Schritt (a) genannte Cyclohexan-Oxidation in Anwesenheit eines Katalysators durchgeführt wird.

8. Verfahren nach einem der Ansprüche 6 bis 7, **dadurch gekennzeichnet, dass** man die in Schritt (b) erhaltene wässrige Phase katalytisch bei einer Temperatur im Bereich von 10 bis 200°C und einem Druck von 1 bis 100 bar hydriert wird.

9. Verfahren nach einem der Ansprüche 6 bis 8, **dadurch gekennzeichnet, dass** man die Veresterung in Schritt (c) mit 1,6-Hexandiol durchführt.

10. Verfahren nach einem der Ansprüche 6 bis 9, **dadurch gekennzeichnet, dass** man für die Veresterung in Schritt c) anstelle mindestens eines Diols mit zwei bis zwölf Kohlenstoffatomen einen Teil des in Schritt (e) gewonnenen Hydrieraustrags verwendet.

11. Verfahren nach einem der Ansprüche 6 bis 10, **dadurch gekennzeichnet, dass** man für die Veresterung in Schritt c) einen Teil des in Schritt (e) gewonnenen Hydrieraustrags verwendet, wobei aus dem gesamten Hydrieraustrag vorher Hoch- und Leichtsieder abgetrennt wurden.

12. Verfahren nach einem der Ansprüche 6 bis 11, **dadurch gekennzeichnet, dass** das für die Hydrierung in Schritt d) verwendete Estergemisch eine Säurezahl von 1 bis 20 mg KOH/g besitzt.

13. Verfahren nach einem der Ansprüchen 6 bis 12, **dadurch gekennzeichnet, dass** man die katalytische Hydrierung des Estergemischs gemäß Schritt d) in der Flüssigphase in mindestens zwei Reaktoren durchführt, wobei der erste Reaktor zur Wärmeabfuhr mit Flüssigkeitsrückführung, der zweite Reaktor zur Vervollständigung des Umsatzes im geraden Durchgang, ohne Flüssigkeitsrückführung betrieben wird.

## Claims

1. A process for hydrogenating oligo- and/or polyesters obtainable by esterifying a dicarboxylic acid solution with a diol or diol mixture, a base selected from the group consisting of lithium alkoxide, potassium alkoxide and sodium alkoxide being metered into the ester mixture obtained, and said hydrogenation being performed by means of a shaped catalyst body whose precursor is preparable by a process in which
(i) an oxidic material comprising copper oxide, aluminum oxide and at least one of the oxides of lanthanum, of tungsten, of molybdenum, of titanium, of zirconium or of iron is provided,
(ii) pulverulent metallic copper, copper flakes, pulverulent cement, graphite or a mixture thereof is added to the oxidic material,
(iii) the mixture resulting from (ii) is shaped to a shaped body,
said oxidic material being obtainable by simultaneous or successive precipitation of the active copper component, of the aluminum component and of the component of at least one of the oxides of lanthanum, of tungsten, of molybdenum, of titanium or of zirconium, and subsequent drying and calcination, and the shaped catalyst body is calcined once again after the shaping in step (iii).

2. The process according to claim 1, wherein the oxidic material comprises
(a) copper oxide with a proportion in the range of 50 ≤ x ≤ 80 and preferably 55 ≤ x ≤ 75% by weight,
(b) aluminum oxide with a proportion in the range of 15 ≤ y ≤ 35 and preferably 20 ≤ y ≤ 30% by weight and
(c) at least one of the oxides of lanthanum, of tungsten, of molybdenum, of titanium or of zirconium with a proportion in the range of 2 ≤ z ≤ 20 and preferably 3 ≤ z ≤ 15% by weight,
based in each case on the total weight of the oxidic material after calcination, where: 80 ≤ x + y + z ≤ 100, especially 95 ≤ x + y + z ≤ 100, cement not being counted among the oxidic material in the above sense.

3. The process according to either of claims 1 and 2, wherein, by means of the addition, the pulverulent metallic copper, the copper flakes, the pulverulent cement or graphite or the mixture thereof is in a proportion in the range from 0.5 to 40% by weight, based on the total weight of the oxidic material, after the first calcination.

4. The process according to any of claims 1 to 3, wherein diols which are used for esterification are alpha,omega-diols having two to twelve carbon atoms or mixtures of these diols.

5. The process according to any of claims 1 to 4, wherein the shaped body obtained from step (iii) is also treated with boiling water and/or steam.

6. A process for preparing 1,6-hexanediol, comprising the following steps:
a) oxidizing cyclohexane with oxygen or oxygen-comprising gases to give mixtures of cyclohexanol, cyclohexanone and carboxylic acids having up to six carbon atoms,
b) reacting the reaction mixture obtained in a) with water and removing the dicarboxylic acid solution from the liquid biphasic reaction mixture,
c) esterifying the dicarboxylic acid solution obtained from b) with an alcohol,
d) catalytically hydrogenating the ester mixture obtained from c) and
e) distilling the hydrogenation output obtained from d),
which comprises
performing the esterification in c) with at least one diol having two to twelve carbon atoms,
a base selected from the group consisting of lithium alkoxide, potassium alkoxide and sodium alkoxide is metered into the ester mixture obtained in c), and
hydrogenating the esterification mixture obtained in c) in the liquid phase in the presence of a shaped catalyst body whose precursor is obtainable by
(i) providing an oxidic material after the first calcination comprising copper oxide, aluminum oxide and at least one of the oxides of lanthanum, of tungsten, of molybdenum, of titanium, of zirconium or of iron,
(ii) adding pulverulent metallic copper, copper flakes, pulverulent cement, graphite or a mixture thereof to the oxidic material from step i),
(iii) shaping the mixture resulting from (ii) to a shaped body and
(iv) second calcination of the shaped body obtained from step (iii).

7. The process according to claim 6, wherein the cyclohexane oxidation specified in step (a) is performed in the presence of a catalyst.

8. The process according to either of claims 6 and 7, wherein the aqueous phase obtained in step (b) is catalytically hydrogenated at a temperature in the range from 10 to 200°C and a pressure of 1 to 100 bar.

9. The process according to any of claims 6 to 8, wherein the esterification in step (c) is performed with 1,6-hexanediol.

10. The process according to any of claims 6 to 9, wherein a portion of the hydrogenation output obtained in step (e) is used instead of at least one diol having two to twelve carbon atoms for the esterification in step c).

11. The process according to any of claims 6 to 10, wherein a portion of the hydrogenation output obtained in step (e) is used for the esterification in step c), high and low boilers having been removed from the overall hydrogenation output beforehand.

12. The process according to any of claims 6 to 11, wherein the ester mixture used for the hydrogenation in step d) possesses an acid number of 1 to 20 mg KOH/g.

13. The process according to any of claims 6 to 12, wherein the catalytic hydrogenation of the ester mixture in step d) is performed in the liquid phase in at least two reactors, the first reactor being operated for removal of heat with liquid recycling, the second reactor for completion of the conversion in straight pass, without liquid recycling.

## Revendications

1. Procédé pour l'hydrogénation d'oligoesters et/ou de polyesters, pouvant être obtenus par estérification d'une solution d'acide dicarboxylique par un diol ou un mélange de diols, le mélange d'esters formé étant additionné en dosant d'une base, choisie dans le groupe constitué par les alcoolates de lithium, de potassium et de sodium, et l'hydrogénation étant réalisée au moyen d'un corps façonné catalytique, dont le précurseur peut être préparé selon un procédé dans lequel
(i) un matériau de type oxyde, comprenant de l'oxyde de cuivre, de l'oxyde d'aluminium et au moins un des oxydes du lanthane, du tungstène, du molybdène, du titane, du zirconium ou du fer, est mis à disposition,
(ii) du cuivre métallique sous forme de poudre, des lamelles de cuivre, du ciment sous forme de poudre, du graphite ou un de leurs mélanges est ajouté au matériau de type oxyde, et
(iii) le mélange résultant de (ii) est façonné en un corps façonné,
le matériau de type oxyde pouvant être obtenu par une précipitation simultanée ou consécutive du composant actif à base de cuivre, du composant à base d'aluminium et du composant à base d'au moins un des oxydes du lanthane, du tungstène, du molybdène, du titane ou du zirconium et séchage et calcination consécutifs et, après le façonnage selon l'étape (iii), le corps façonné catalytique est à nouveau calciné.

2. Procédé selon la revendication 1, **caractérisé en ce que** le matériau de type oxyde comprend
(a) de l'oxyde de cuivre en une proportion dans la plage de 50 ≤ x ≤ 80, de préférence de 55 ≤ x ≤ 75% en poids,
(b) de l'oxyde d'aluminium en une proportion dans la plage 15 ≤ y ≤ 35, de préférence 20 ≤ y ≤ 30% en poids et
(c) au moins un des oxydes du lanthane, du tungstène, du molybdène, du titane ou du zirconium en une proportion dans la plage de 2 ≤ z ≤ 20, de préférence de 3 ≤ z ≤ 15% en poids,
à chaque fois par rapport au poids total du matériau de type oxyde après calcination, avec 80 ≤ x + y + z ≤ 100, en particulier 95 ≤ x + y + z ≤ 100, le ciment n'étant pas inclus dans le calcul du matériau de type oxyde dans le sens ci-dessus.

3. Procédé selon l'une quelconque des revendications 1 ou 2, **caractérisé en ce que**, par l'addition, le cuivre métallique sous forme de poudre, les lamelles de cuivre, le ciment sous forme de poudre ou le graphite ou leur mélange représente une proportion dans la plage allant de 0,5 à 40 % en poids, par rapport au poids total du matériau de type oxyde après la première calcination.

4. Procédé selon l'une quelconque des revendications 1 à 3, **caractérisé en ce que** les diols utilisés pour l'estérification sont des alpha,omega-diols comprenant deux à douze atomes de carbone ou des mélanges de ces diols.

5. Procédé selon l'une quelconque des revendications 1 à 4, **caractérisé en ce que** le corps façonné obtenu de l'étape (iii) est encore traité à l'eau bouillante et/ou à la vapeur.

6. Procédé pour la préparation de 1,6-hexanediol, comprenant les étapes suivantes
a) oxydation de cyclohexane avec de l'oxygène ou des gaz contenant de l'oxygène en mélanges de cyclohexanol, de cyclohexanone et d'acides carboxyliques comprenant jusqu'à six atomes de carbone,
b) conversion du mélange réactionnel obtenu selon a) avec de l'eau et séparation de la solution d'acide dicarboxylique du mélange réactionnel liquide à deux phases,
c) estérification de la solution d'acide dicarboxylique obtenue de b) avec un alcool,
d) hydrogénation catalytique du mélange d'esters obtenu de c) et
e) distillation du produit d'hydrogénation obtenu de d),
**caractérisé en ce qu'**on
réalise l'estérification dans c) avec au moins un diol comprenant deux à douze atomes de carbone,
ajoute en dosant au mélange d'esters obtenu dans c) une base choisie dans le groupe constitué par les alcoolates de lithium, de potassium et de sodium, et hydrogène le mélange d'estérification obtenu dans c) en phase liquide en présence d'un corps façonné catalytique, donc le précurseur peut être obtenu par
(i) la mise à disposition d'un matériau de type oxyde contenant après une première calcination de l'oxyde de cuivre, de l'oxyde d'aluminium et au moins un des oxydes du lanthane, du tungstène, du molybdène, du titane, du zirconium ou du fer,
(ii) l'addition de cuivre métallique sous forme de poudre, de lamelles de cuivre, de ciment sous forme de poudre, de graphite ou d'un de leurs mélanges au matériau de type oxyde de l'étape i)
(iii) façonnage du mélange résultant de (ii) en un corps façonné et
(iv) deuxième calcination du corps façonné obtenu de l'étape (iii).

7. Procédé selon la revendication 6, **caractérisé en ce que** l'oxydation de cyclohexane mentionnée dans l'étape (a) est réalisée en présence d'un catalyseur.

8. Procédé selon l'une quelconque des revendications 6 à 7, **caractérisé en ce qu'**on hydrogène la phase aqueuse obtenue dans l'étape (b) catalytiquement à une température dans la plage de 10°C à 200°C et à une pression dans la plage de 1 à 100 bars.

9. Procédé selon l'une quelconque des revendications 6 à 8, **caractérisé en ce qu'**on réalise l'estérification dans l'étape (c) avec du 1,6-hexanediol.

10. Procédé selon l'une quelconque des revendications 6 à 9, **caractérisé en ce qu'**on utilise pour l'estérification dans l'étape c), au lieu d'au moins un diol comprenant deux à douze atomes de carbone, une partie du produit d'hydrogénation obtenu dans l'étape (e).

11. Procédé selon l'une quelconque des revendications 6 à 10, **caractérisé en ce qu'**on utilise pour l'estérification dans l'étape c) une partie du produit d'hydrogénation obtenu dans l'étape (e), des fractions de bas point d'ébullition et de point d'ébullition élevé étant séparées du produit d'hydrogénation total.

12. Procédé selon l'une quelconque des revendications 6 à 11, **caractérisé en ce que** le mélange d'esters utilisé pour l'hydrogénation dans l'étape d) présente un indice d'acide de 1 à 20 mg de KOH/g.

13. Procédé selon l'une quelconque des revendications 6 à 12, **caractérisé en ce qu'**on réalise l'hydrogénation catalytique du mélange d'esters selon l'étape d) en phase liquide dans au moins deux réacteurs, le premier réacteur étant exploité pour l'évacuation de chaleur avec un recyclage de liquide, le deuxième réacteur étant exploité pour compléter la conversion en passage direct, sans recyclage de liquide.
